# EUROPEAN PATENT SPECIFICATION

(11) **EP 3 470 389 B1**
(45) Date of publication and mention of the grant of the patent: **18.05.2022**
(21) Application number: 16897136.4
(22) Date of filing: 30.03.2016
(51) Int. Cl.: C25B 1/04, C25B 3/07, C25B 3/26, C25B 9/19, C25B 9/70, C25B 15/08

(54) **FORMIC ACID PREPARATION METHOD**
AMEISENSÄUREHERSTELLUNGSVERFAHREN
PROCÉDÉ DE PRÉPARATION D'ACIDE FORMIQUE

(30) Priority: 29.03.2016 KR 20160037986
(43) Date of publication of application: 17.04.2019
(73) Proprietor: Techwin Co., Ltd., Cheongju-si, Chungcheongbuk-do 28580 (KR)
(72) Inventor: JUNG, Boong Ik, Cheongju-si Chungcheongbuk-do 28457 (KR); KIM, Jung Sik, Heungdeok-gu Cheongju-si Chungcheongbuk-do 28424 (KR); SHIN, Hyun Su, Daedeok-gu Daejeon 34390 (KR); HYUN, Sun Taek, Heungdeok-gu Cheongju-si Chungcheongbuk-do 28426 (KR)
(74) Representative: Krauns, Christian
(86) International application number: PCT/KR2016/003237
(87) International publication number: WO 2017/171115

(56) References cited:
- WO-A1-2014/042782
- WO-A2-2014/042781
- JP-A- 2009 511 740
- KR-A- 20150 055 033
- FERRER ET AL: 'Formic Acid Regeneration by Electromembrane Processes' JOURNAL OF MEMBRANE SCIENCE vol. 280, no. ISSUES, 06 March 2006, pages 509 - 516, XP005544303

## Description

### Technical Field

The present invention relates to a method for producing formic acid, and more particularly to a method for producing formic acid, in which formic acid may be produced by minimizing the additional introduction of an acid, such as hydrochloric acid or sulfuric acid, which is added to acidify formate into formic acid.

### Background Art

Gases that affect global warming are referred to as greenhouse gases. These greenhouse gases include carbon dioxide, methane and CFCs. In a report released in 2010, global carbon dioxide emissions amounted to about 3.3 billion tons, which is an increase of about 45% compared to carbon dioxide emissions in 1990. Korea's carbon dioxide emissions are 7^{th} highest in the world, and the rate of increase thereof is 3^{rd} highest in the world. In order to decrease carbon dioxide emissions, developed countries have introduced emissions trading and carbon taxes.

Methods of lowering carbon dioxide emissions include capture, storage and conversion processes. A carbon dioxide capture and storage (CCS) technique is a technology for isolating carbon dioxide emitted from sources such as power plants, steel mills and cement plants from the atmosphere. Carbon dioxide thus captured may be stored in the ocean, in the ground, or on the ground. However, carbon dioxide storage methods have problems such as marine ecosystem problems, storage site selection problems, and carbon dioxide transport problems. Therefore, many methods of converting carbon dioxide in an electrochemical manner have been studied.

The conversion of carbon dioxide using electrical energy enables the conversion into various organic compounds such as carbon monoxide, formic acid, methanol, methane and the like through an electrode reaction at room temperature under atmospheric pressure depending on the kind of electrode material and on the reaction conditions.

In particular, formic acid may be generated through a two-electron reaction that uses less energy than other conversion targets. Recently, thorough research into formic acid is ongoing. Formic acid is used to keep food fresh when raising livestock, and is also used in a small amount as a food preservative. Furthermore, formic acid may be used as fuel for formic acid fuel cells.

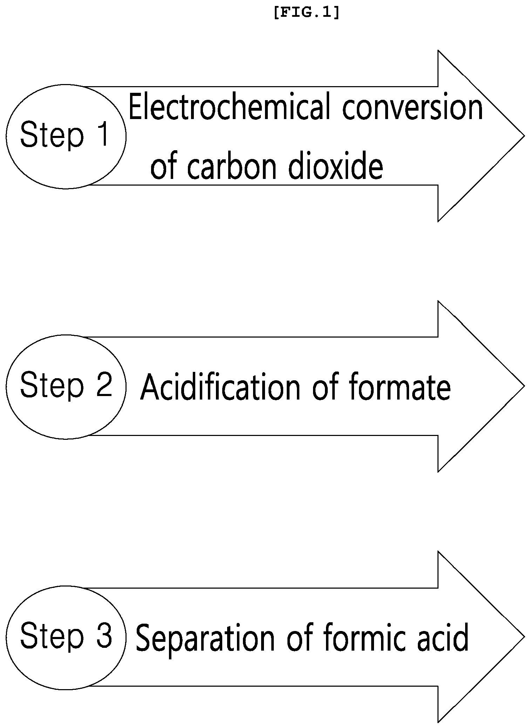

As the related literature on the production of formic acid using such an electrolysis process, Korean Patent No. 10-1372532 discloses "Electrochemical reduction method of carbon dioxide using a solution containing potassium sulfate". As shown in FIG. 1, the electrochemical reduction method of carbon dioxide includes converting carbon dioxide into formate in an electrochemical manner (step 1), introducing sulfuric acid in order to acidify formate (step 2), and separating formic acid (step 3).

In step 1, carbon dioxide is converted into formate (e.g. HCOOK or HCOONa) through an electrode reaction in an electrolytic bath. In step 2, sulfuric acid (H₂SO₄) or hydrochloric acid (HCl) is added. Thereby, the formate is acidified into formic acid (HCOOH). In step 3, the formic acid (HCOOH) is concentrated and separated.

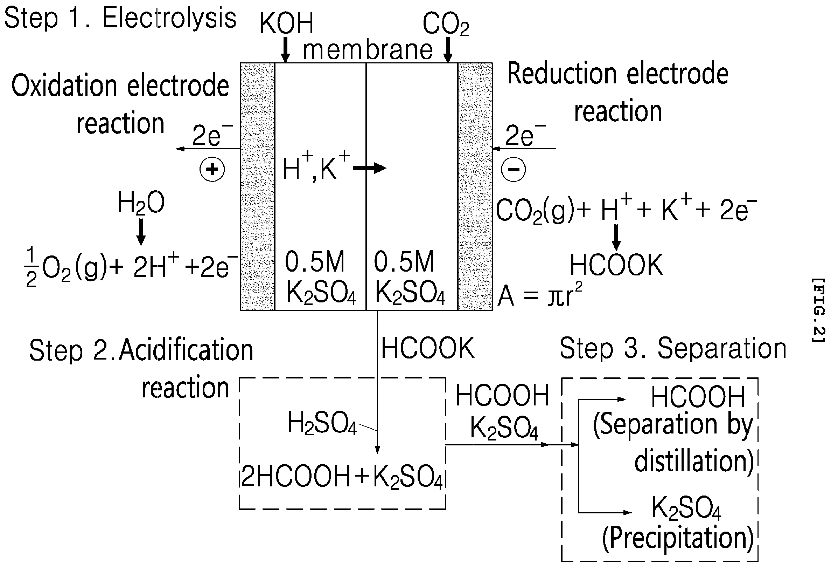

FIG. 2 shows an electrochemical conversion process of carbon dioxide using a solution containing potassium sulfate according to the conventional technique. In step 1, oxidation of water in an oxidation electrode unit generates oxygen and a hydrogen ion (H⁺). K⁺ and H⁺ in solution phase are transferred to a reduction electrode unit through a cation membrane. In the reduction electrode unit, carbon dioxide is converted into formate (HCOOK) through the electrode reaction. In this procedure, KOH is continuously supplied to the oxidation electrode unit in order to control an ion balance. In step 2, when 2 mol of formic acid is produced using sulfuric acid (H₂SO₄), 1 mol of potassium sulfate is produced. In step 3, formic acid is concentrated and potassium sulfate is precipitated, thereby separating formic acid from potassium sulfate.

The conventional technique has the following problems.

First, KOH has to be continuously supplied to the oxidation electrode unit.

Second, in order to acidify formate into formic acid, a chemical material such as hydrochloric acid or sulfuric acid has to be introduced, and an additional device therefor has to be provided.

### Disclosure

### Technical Problem

Accordingly, the present invention is intended to provide a method for producing formic acid, in which formic acid may be produced by minimizing the addition of a chemical material such as hydrochloric acid or sulfuric acid upon conversion of formate into formic acid.

### Technical Solution

The problem of the present invention is solved by a method of producing formic acid according to the independent claim 1, while the dependent claims refer to further advantageous developments of the present invention.

### Advantageous Effects

According to the present invention, the method for producing formic acid can exhibit the following effects.

First, the additional supply of a chemical material in order to convert formate into formic acid can be minimized.

Second, an alkali metal or alkaline earth metal cation necessary for formate formation can be supplied to an additional electrolytic bath by a circulation loop without additional supply thereof.

### Description of Drawings

FIG. 1 is a flowchart showing a typical process of producing formic acid;
FIG. 2 shows the configuration of a conventional formic acid production apparatus and the operation thereof;
FIG. 3 shows the configuration of a formic acid production apparatus according to an example useful for the understanding of the present invention;
FIG. 4 is a flowchart showing a process of producing formic acid using the formic acid production apparatus of FIG. 3;
FIG. 5 shows the configuration of a formic acid production apparatus according to another example useful for the understanding of the present invention; and
FIG. 6 is a flowchart showing a process of producing formic acid using the formic acid production apparatus of FIG. 5.

### Best Mode

Hereinafter, a detailed description will be given of the present invention with reference to the appended drawings.

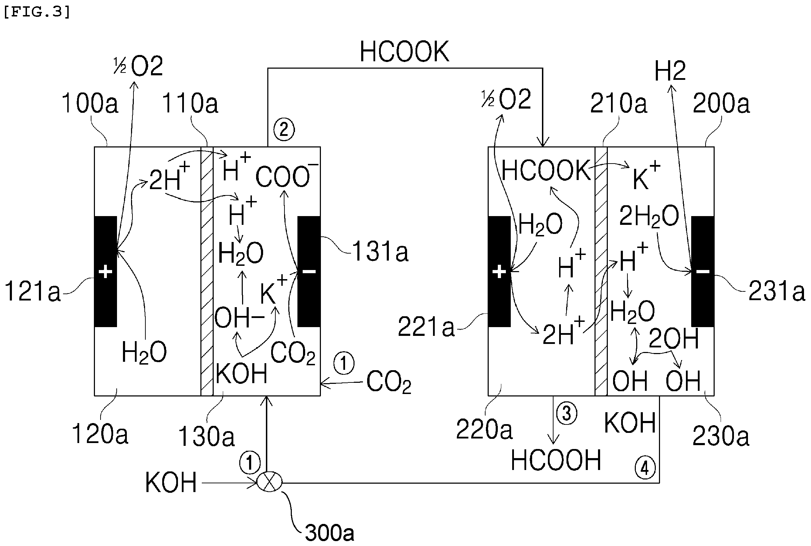

As shown in FIG. 3, a formic acid production apparatus according to an example useful for the understanding of the present invention is configured to include a first diaphragm electrolytic bath 100a and a second diaphragm electrolytic bath 200a. The first diaphragm electrolytic bath 100a includes a first anode chamber 120a and a first cathode chamber 130a, which are disposed to face each other with a first diaphragm 110a interposed therebetween, and the first anode chamber 120a is provided with a first anode 121a and the first cathode chamber 130a is provided with a first cathode 131a. The second diaphragm electrolytic bath 200a includes a second anode chamber 220a and a second cathode chamber 230a, which are disposed to face each other with a second diaphragm 210a interposed therebetween, and the second anode chamber 220a is provided with a second anode 221a and the second cathode chamber 230a is provided with a second cathode 231a. The first diaphragm 110a and the second diaphragm 210a of the first diaphragm electrolytic bath 100a and the second diaphragm electrolytic bath 200a are preferably cation exchange membranes.

The first diaphragm electrolytic bath 100a functions to produce formate from carbon dioxide that is supplied. The second diaphragm electrolytic bath 200a is configured such that the formate supplied from the first diaphragm electrolytic bath 100a is converted into formic acid, thus yielding a target product, and also such that an alkali metal or alkaline earth metal electrolyte is generated and supplied to the first diaphragm electrolytic bath 100a.

The alkali metal or alkaline earth metal electrolyte may be an electrolyte obtained by combining a cation, such as an alkali metal including sodium (Na⁺) or potassium (K⁺), an alkaline earth metal including calcium (Ca²⁺) or magnesium (Mg²⁺), or ammonium (NH₄⁺) with an anion such as a hydroxide ion (OH⁻) or a sulfate ion (SO₄²⁻).

Also, the formate of the present invention is provided in the form in which the formic acid ion (HCOO⁻) is combined with the above-listed cation, and examples thereof include chemical products, such as potassium formate (HCOOK), sodium formate (HCOONa), calcium formate ((HCOO)₂Ca), magnesium formate ((HCOO)₂Mg), and ammonium formate (HCOONH₄).

In the following detailed description of the present invention, the alkali metal or alkaline earth metal electrolyte includes potassium hydroxide (KOH) and potassium sulfate (K₂SO₄) and the formate includes potassium formate (HCOOK) produced from the above electrolyte, but the present invention is not limited to these compounds.

According to an embodiment of the present invention as shown in FIG. 3, formic acid is produced in a manner in which water (H₂O) is supplied to the first anode chamber 120a of the first diaphragm electrolytic bath 100a, and both potassium hydroxide (KOH) and carbon dioxide (CO₂) are simultaneously supplied to the first cathode chamber 130a (refer to mark ①). Here, a direction-switching valve 300a is set such that potassium hydroxide (KOH) is supplied to the first cathode chamber 130a from an external supply unit (not shown).

When an electrolyte solution is supplied to the first diaphragm electrolytic bath and direct-current (DC) power is applied to the first anode 121a and the first cathode 131a by means of respective DC power supplies (not shown), individual electrolysis reactions occur at the first anode 121a and the first cathode 131a.

At the first anode 121a, water (H₂O) is converted into oxygen (O₂) and a hydrogen ion (H⁺) through the electrolysis reaction represented by Scheme (1) below, and at the first cathode 131a, carbon dioxide (CO₂) is converted into a formic acid ion (HCOO⁻) through the electrolysis reaction represented by Scheme (2) below.

H₂O → ½O₂ + 2H⁺ + 2e⁻ ...................................................... (1)

CO₂ + H₂O + 2e⁻ → HCOO⁻ + OH⁻ .................................... (2)

The hydrogen ion (H⁺) generated at the first anode 121a is transferred to the first cathode chamber 130a through the first diaphragm 110a from the first anode chamber 120a.

The hydrogen ion (H⁺) passed through the first diaphragm 110a is combined with the hydroxide ion (OH-) generated through the electrolysis reaction and the hydroxide ion (OH⁻) of potassium hydroxide (KOH) supplied to the first cathode chamber and is thus converted into water (H₂O), as represented by Scheme (3) below, and the potassium cation (K⁺) remaining after conversion into water is combined with the formic acid ion (HCOO⁻) generated through the electrolysis reaction, thus yielding formate, that is, potassium formate (HCOOK).

H⁺ + OH⁻ → H₂O ............................................. (3)

Here, water (H₂O) supplied to the first anode chamber 120a may be further added with an electrolyte such as potassium hydroxide (KOH) in order to decrease electrical resistance upon electrolysis.

When the electrolyte such as potassium hydroxide (KOH) is further added in this way, a neutralization reaction occurs in a manner in which the hydrogen ion (H⁺) generated at the first anode 121a is combined with the hydroxide ion (OH⁻) of potassium hydroxide (KOH) to afford water (H₂O), and the remaining potassium ion (K⁺) is transferred to the first cathode chamber 130a through the first diaphragm 110a. If the electrolyte supplied to the first anode chamber 120a is potassium sulfate (K₂SO₄), sulfuric acid (H₂SO₄) is formed through the reaction with a hydrogen ion (H⁺), rather than the neutralization reaction, and similarly, the potassium ion (K⁺) is transferred to the first cathode chamber 130a through the first diaphragm 110a.

The type of reaction product in the first anode chamber 120a may vary depending on the supply and kind of electrolyte, but the overall ion balance and the process of producing potassium formate (HCOOK) are the same.

Next, potassium formate (HCOOK) generated in the first cathode chamber 130a of the first diaphragm electrolytic bath 100a is supplied to the second anode chamber 220a of the second diaphragm electrolytic bath 200a (refer to mark (2)), and water (H₂O) is supplied to the second cathode chamber 230a.

In this way, when the electrolyte solution is supplied to the second diaphragm electrolytic bath 200a and DC power is applied to the second anode 221a and the second cathode 231a by means of respective DC power supplies (not shown), a water decomposition reaction for generating a hydrogen ion (H⁺) is carried out at the second anode 221a, through the electrolysis reaction represented by Scheme (1), and a water decomposition reaction for generating a hydroxide ion (OH⁻) is carried out at the second cathode 231a, through the electrolysis reaction represented by Scheme (4) below.

2H₂O + 2e⁻ → H₂ + 2OH⁻ ...................................................... (4)

In the second anode chamber 220a, the hydrogen ion (H⁺) generated at the second anode 221a is combined with potassium formate (HCOOK) prepared in the first cathode chamber 130a and supplied therefrom to yield formic acid (HCOOH) (Scheme (5)), thereby obtaining a target product, that is, formic acid (HCOOH) (refer to mark ③).

HCOOK + H⁺ → HCOOH + K⁺ ................................................... (5)

Here, an evaporator or a separator (not shown) may be further disposed downstream of the formic acid production apparatus in order to adjust the desired product concentration and purity in the produced formic acid (HCOOH).

The hydrogen ion (H⁺) and the potassium ion (K⁺) remaining after conversion into formic acid (HCOOH) are transferred to the second cathode chamber 230a through the second diaphragm 210a, and the transferred potassium ion (K⁺) is combined with the hydroxide ion (OH⁻) generated via the electrolysis reaction of the second cathode 231a, thus forming potassium hydroxide (KOH). As such, a hydrogen ion (H⁺) and a hydroxide ion (OH⁻), which do not participate in the reaction, are combined with each other to give water (H₂O), whereby the overall ion balance is controlled.

Here, water (H₂O) supplied to the second cathode chamber 230a may be further added with an electrolyte such as potassium hydroxide (KOH) in order to decrease electrical resistance upon electrolysis, and the supply of such an electrolyte is set so as not to affect the overall ion balance.

The direction-switching valve 300a operates such that potassium hydroxide (KOH) generated in the second cathode chamber 230a of the second diaphragm electrolytic bath 200a is transferred to the first cathode chamber 130a of the first diaphragm electrolytic bath 100a. Accordingly, potassium hydroxide (KOH) generated in the second cathode chamber 230a of the second diaphragm electrolytic bath 200a is transferred to the first cathode chamber 130a of the first diaphragm electrolytic bath 100a, and thus potassium hydroxide (KOH) necessary for the second diaphragm electrolytic bath 200a may be regenerated even without the additional supply of potassium hydroxide (KOH) (refer to mark ①), whereby potassium formate and formic acid may be produced so as to control the overall ion balance (refer to mark ④).

However, in the actual process, some electrolyte loss, caused by current efficiency, movement of the material through the membrane and the rate thereof, and loss due to transport of the solution and during the reaction, may be compensated for through additional supply.

The present embodiment may be further configured such that water discharged from the first anode chamber 120a is supplied to the second cathode chamber 230a. This configuration may decrease water consumption and may slightly lower the electrical resistance of the electrolyte solution supplied to each electrolytic bath.

Also, the present embodiment may be further configured such that a portion of the potassium hydroxide (KOH) solution supplied to the first cathode chamber 130a from the second cathode chamber 230a is supplied to the first anode chamber 120a. This configuration may decrease the electrical resistance of the electrolyte solution supplied to the first anode chamber 120a.

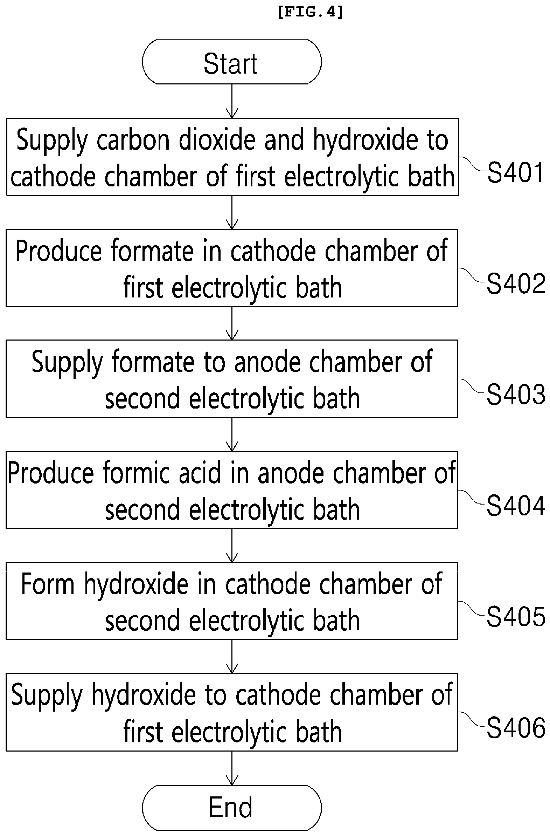

FIG. 4 is a flowchart showing the process of producing formic acid using the formic acid production apparatus of FIG. 3. First, carbon dioxide (CO₂) and potassium hydroxide (KOH) are supplied to the first cathode chamber 130a of the first electrolytic bath 100a. Here, water is supplied to the first anode chamber 120a of the first electrolytic bath 100a (S401).

In the first anode chamber 120a, water (H₂O) is electrolyzed, and thus a hydrogen ion (H⁺) is transferred to the first cathode chamber 130a. The hydrogen ion (H⁺) is subjected to a neutralization reaction with potassium hydroxide (KOH), thus generating a potassium ion (K⁺). As such, a formic acid ion (HCOO⁻) is generated via electrolysis of carbon dioxide (CO₂) in the first cathode chamber 130a, and is combined with the potassium ion (K⁺) to yield potassium formate (HCOOK) (S402).

Potassium formate (HCOOK) generated in the first cathode chamber 130a of the first electrolytic bath 100a is supplied to the second anode chamber 220a of the second electrolytic bath 200a (S403).

Potassium formate (HCOOK) is combined with the hydrogen ion (H⁺) generated by the electrolysis of water (H₂O) in the second anode chamber 220a of the second electrolytic bath 200a, thus producing formic acid (HCOOH) (S404).

The hydrogen ion (H⁺) and the potassium ion (K⁺), remaining after production of formic acid (HCOOH), are transferred to the second cathode chamber 230a through the second diaphragm 210a from the second anode chamber 220a, and are combined with two hydroxyl groups (OH⁻) generated by the electrolysis of water in the second cathode chamber 230a of the second electrolytic bath 200a and thus converted into water (H₂O) and potassium hydroxide (KOH), respectively (S405).

Potassium hydroxide (KOH) generated in the second cathode chamber 230a of the second electrolytic bath 200a is supplied to the first cathode chamber 130a of the first electrolytic bath 110a (S406).

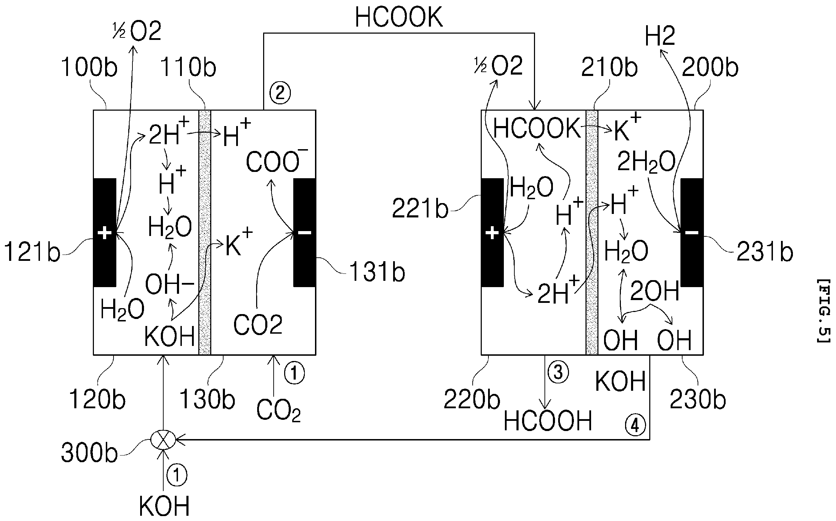

FIG. 5 shows the configuration of a formic acid production apparatus according to another example useful for the understanding of the present invention.

Similar to the aforementioned example useful for the understanding of the present invention, a first diaphragm electrolytic bath 100b and a second diaphragm electrolytic bath 200b are included. The first diaphragm electrolytic bath 100b includes a first anode chamber 120b having a first anode 121b provided therein and a first cathode chamber 130b having a first cathode 131b provided therein, which are disposed to face each other with a first diaphragm 110b interposed therebetween, and the second diaphragm electrolytic bath 200b includes a second anode chamber 220b having a second anode 221b provided therein and a second cathode chamber 230b having a second cathode 231b provided therein, which are disposed to face each other with a second diaphragm 210b interposed therebetween. Unlike the example of FIG. 3, the present example is configured such that an alkali metal or alkaline earth metal electrolyte is supplied to the first anode chamber 120b of the first diaphragm electrolytic bath 100b.

The alkali metal or alkaline earth metal electrolyte may be an electrolyte obtained by combining a cation, such as an alkali metal including sodium (Na⁺) or potassium (K⁺), an alkaline earth metal including calcium (Ca²⁺) or magnesium (Mg²⁺), or ammonium (NH₄⁺) with an anion such as a hydroxide ion (OH⁻) or a sulfate ion (SO₄²⁻).

Also, the formate of the present invention is provided in the form in which the formic acid ion (HCOO⁻) is combined with the above-listed cation, and examples thereof include chemical products, such as potassium formate (HCOOK), sodium formate (HCOONa), calcium formate ((HCOO)₂Ca), magnesium formate ((HCOO)₂Mg), and ammonium formate (HCOONH₄).

In the following detailed description of the present invention, the alkali metal or alkaline earth metal electrolyte includes potassium hydroxide (KOH) and potassium sulfate (K₂SO₄) and the formate includes potassium formate (HCOOK) produced from the above electrolyte, but the present invention is not limited to these compounds as mentioned above.

According to another example useful for the understanding of the present invention as shown in FIG. 5, a direction-switching valve 300b is set such that potassium hydroxide (KOH) is supplied to the first anode chamber 120b of the first electrolytic bath 100b from an external supply unit (not shown). The basic configuration and operation of the second diaphragm electrolytic bath 200b are similar to the embodiment of FIG. 3 described above.

A potassium hydroxide (KOH) aqueous solution is supplied to the first anode chamber 120b, and carbon dioxide (CO₂) is supplied together with an electrolyte solution to the first cathode chamber 130b (refer to the mark). When the electrolyte solution is supplied to the first diaphragm electrolytic bath and DC power is applied to the first anode 121b and the first cathode 131b by means of respective DC power supplies (not shown), individual electrolysis reactions occur at the first anode 121b and the first cathode 131b.

At the first anode 121b, water (H₂O) is electrolyzed to thus generate oxygen (O₂) and a hydrogen ion (H⁺), as represented by Scheme (1), and at the first cathode 131b, carbon dioxide (CO₂) is converted into a formic acid ion (HCOO⁻) through the electrolysis reaction represented by Scheme (2).

The hydrogen ion (H⁺) generated at the first anode 121b is subjected to a neutralization reaction with the hydroxide ion (OH⁻) of potassium hydroxide (KOH) introduced to the first anode chamber 120b, as represented by Scheme (3), thus generating water and a potassium ion (K⁺).

The potassium ion (K⁺) and the hydrogen ion (H⁺) thus generated are transferred to the first cathode chamber 130b through the first diaphragm 110b, and the potassium ion (K⁺) and the hydrogen ion (H⁺) thus transferred are combined with the formic acid ion (HCOO⁻) and the hydroxide ion (OH⁻) generated at the first cathode 131b and thus converted into potassium formate (HCOOK) or potassium hydroxide (KOH) and water (H₂O), thereby producing formate (refer to the mark).

Formate (HCOOK) and potassium hydroxide (KOH) generated in the first cathode chamber 130b of the first electrolytic bath 100b are supplied to the second anode chamber 220b of the second electrolytic bath 200b (refer to the mark), and water (H₂O) is supplied to the second cathode chamber 230b.

When the electrolyte solution is supplied to the second diaphragm electrolytic bath 200b and DC power is applied to the second anode 221b and the second cathode 231b by means of respective DC power supplies (not shown), a water decomposition reaction for generating a hydrogen ion (H⁺) through the electrolysis reaction represented by Scheme (1) is carried out at the second anode 221b and a water decomposition reaction for generating a hydroxide ion (OH⁻) through the electrolysis reaction represented by Scheme (4) is carried out at the second cathode 231b.

In the second anode chamber 220b, the hydrogen ion (H⁺) generated at the second anode 221b is combined with potassium formate (HCOOK) prepared in the first cathode chamber 130b and supplied therefrom and is thus converted into formic acid (HCOOH) (Scheme (5)), thereby obtaining a target product, that is, formic acid (HCOOH) (refer to the mark).

Here, an evaporator or a separator (not shown) may be further disposed downstream of the formic acid production apparatus in order to adjust the desired product concentration and purity in the produced formic acid (HCOOH).

The potassium ion (K⁺) or the hydrogen ion (H⁺), resulting from neutralization of the potassium ion (K⁺), potassium hydroxide (KOH) and hydrogen ion (H⁺) remaining after conversion into formic acid (HCOOH), is transferred to the second cathode chamber 230b through the second diaphragm 210b, and the transferred potassium ion (K⁺) is combined with the hydroxide ion (OH⁻) generated via the electrolysis reaction of the second cathode 231b, thus forming potassium hydroxide (KOH). As such, a hydrogen ion (H⁺) and a hydroxide ion (OH⁻), which do not participate in the reaction, are combined with each other to give water (H₂O), whereby the overall ion balance is controlled.

The direction-switching valve 300b operates such that potassium hydroxide (KOH) generated in the second cathode chamber 230b of the second diaphragm electrolytic bath 200b is transferred to the first anode chamber 120b of the first diaphragm electrolytic bath 100b. Accordingly, potassium hydroxide (KOH) generated in the second cathode chamber 230b of the second diaphragm electrolytic bath 200b is transferred to the first anode chamber 120b of the first diaphragm electrolytic bath 100b and thus potassium hydroxide (KOH) necessary for the second diaphragm electrolytic bath 200a may be regenerated even without additional supply of potassium hydroxide (KOH) (refer to the mark), whereby potassium formate and formic acid may be produced so as to control the overall ion balance (refer to the mark).

However, in the actual process, some electrolyte loss, caused by current efficiency, movement of the material through the membrane and the rate thereof, and loss due to transport of the solution and during the reaction, may be compensated for through additional supply.

In the above embodiment, the material balance between the hydrogen ion (H⁺) and the potassium ion (K⁺) may vary depending on the initial amount of potassium hydroxide (KOH) that is added, but in all cases, the material balance is controlled through individual electrolysis and electrolyte reactions, whereby the ion balance may be maintained.

The present embodiment may be further configured such that water discharged from the first anode chamber 120b is supplied to the second cathode chamber 230b, whereby the alkali metal or alkaline earth metal electrolyte solution may be circulated to the first anode chamber and the second cathode chamber. This configuration may decrease water consumption and electrical resistance of the electrolyte solution supplied to each electrolytic bath, thus realizing a reduction in power consumption.

Also, the present embodiment may be further configured such that the electrolyte is further supplied to the solution supplied to the first cathode chamber 130b. Also, the present embodiment may be further configured such that a portion of the alkali metal or alkaline earth metal electrolyte solution supplied from the second cathode chamber 230b to the first anode chamber 120b is supplied to the first cathode chamber 130b. This configuration may decrease the electrical resistance of the electrolyte solution supplied to the first cathode chamber 130b.

Still another application embodiment of the present embodiment with the configuration of FIG. 5 is as follows.

Water (H₂O) and potassium sulfate (K₂SO₄) are supplied to the first anode chamber 120b, and carbon dioxide (CO₂) is supplied together with the electrolyte solution to the first cathode chamber 130b, whereby an electrolysis reaction is carried out in the first diaphragm electrolytic bath 100b, as in the above embodiment.

In the first anode chamber 120b, the hydrogen ion (H⁺) reacts with potassium sulfate (K₂SO₄) to thus generate sulfuric acid (H₂SO₄) and a potassium ion (K⁺). The potassium ion (K⁺) is transferred to the first cathode chamber 120b through the first diaphragm 110b, as in the above embodiment, thus generating potassium formate (HCOOK).

The present application embodiment may be further configured such that water discharged from the first anode chamber 120b is supplied to the second cathode chamber 230b as described above, and thus the sulfuric acid (H₂SO₄) solution generated in the first anode chamber 120b is supplied to the second cathode chamber 230b.

In the second diaphragm electrolytic bath 200b, potassium formate (HCOOK) is supplied to the second anode chamber 220b and thus formic acid (HCOOH) is produced as in the above embodiment, and the potassium ion (K+) is transferred to the second cathode chamber 230b through the second diaphragm 210b from the second anode chamber 220b.

Here, sulfuric acid (H₂SO₄) supplied to the second cathode chamber 230b is subjected to neutralization with the hydroxide ion (OH⁻) generated at the second cathode 231b to thus form the sulfuric acid ion (SO₄²⁻), which is then allowed to react with the potassium ion (K⁺) supplied through the second diaphragm and thus converted into potassium sulfate (K₂SO₄), which is then supplied again to the first anode chamber 120b.

Although the neutralization or acid or base generation may vary depending on the ion composition or concentration of the alkali metal or alkaline earth metal electrolyte that is supplied, these embodiments have the same characteristics in terms of the formation of products through electrolysis and the production of formate and formic acid through transfer of ionic materials by means of the diaphragms, or the supply of the alkali metal or alkaline earth metal electrolyte solution, which is added to produce formate, through regeneration in the second diaphragm electrolytic bath 200b.

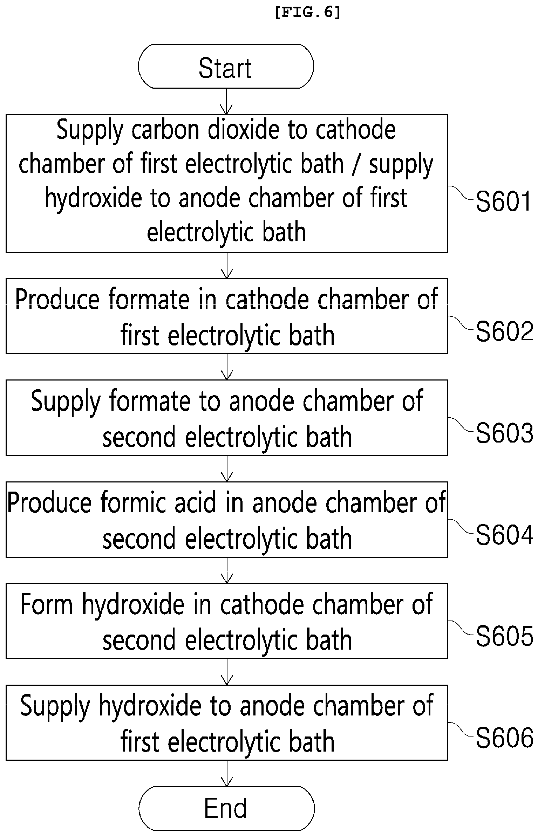

FIG. 6 is a flowchart showing the process of producing formic acid using the formic acid production apparatus according to another embodiment of the present invention as shown in FIG. 5. First, water (H₂O) and potassium hydroxide (KOH) are supplied to the first anode chamber 120b of the first electrolytic bath 100b. Here, carbon dioxide (CO₂) is supplied to the first cathode chamber 130b (S601).

In the first anode chamber 120b, a hydrogen ion (H⁺), among the two hydrogen ions (H⁺) generated by electrolyzing water (H₂O), is combined with the hydroxyl group (OH⁻) of potassium hydroxide (KOH), thus generating water (H₂O) and a potassium ion (K⁺). The remaining hydrogen ion (H⁺) and the potassium ion (K⁺) are transferred to the first cathode chamber 130b through the first diaphragm 110b. Carbon dioxide (CO₂) fed to the first cathode chamber 130b is electrolyzed into a formic acid ion (HCOO⁻ ) and a hydroxide ion (OH⁻). As such, the hydrogen ion (H⁺) transferred through the first diaphragm 110b is combined with the hydroxide ion (OH⁻) to give water (H₂O), and the potassium ion (K⁺) is combined with the formic acid ion (HCOO⁻) to yield potassium formate (HCOOK) (S602).

The formate (HCOOK) obtained in the first cathode chamber 130b of the first electrolytic bath 100b is supplied to the second anode chamber 220b of the second electrolytic bath 200b (S603).

The formate (HCOOK) is combined with one of two hydrogen ions (H⁺) generated by electrolyzing water (H₂O) in the second anode chamber 220b of the second electrolytic bath 200b, thus producing formic acid (HCOOH) (S604).

The remaining hydrogen ion (H⁺) and the potassium ion (K⁺) are transferred to the second cathode chamber 230b through the second diaphragm 210b from the second anode chamber 220b, and are then combined with two hydroxyl groups (OH⁻) generated by electrolysis of water at the second cathode 231b and thus converted into water (H₂O) and potassium hydroxide (KOH), respectively (S605).

The potassium hydroxide (KOH) generated in the second cathode chamber 230b of the second electrolytic bath 200b is supplied to the first anode chamber 120b of the first electrolytic bath 110b (S606).

The above embodiment is merely exemplary, and does not limit the configurations and contents of the present invention, and the scope of the present invention is defined by the claims.

As described hereinbefore, the method for producing formic acid according to the present invention obviate the additional supply of sulfuric acid in order to oxidize formate into formic acid. In the present invention, the second electrolytic bath is further provided, whereby formate may be converted into formic acid through oxidation in the anode chamber of the second electrolytic bath. Moreover, the hydroxide generated in the cathode chamber of the second electrolytic bath is transferred to the first electrolytic bath, and thus there is no need to additionally supply the hydroxide to the first electrolytic bath after the initial formation of formate in the first electrolytic bath.

## Claims

1. A method of producing formic acid through electrical reduction of carbon dioxide, comprising:
a first electrolysis step of producing a formate from carbon dioxide that is supplied, wherein the first electrolysis step is carried out in a first diaphragm electrolytic bath (100a) of an apparatus for producing formic acid, wherein the first diaphragm electrolytic bath (100a) includes a first cathode chamber (130a) and a first anode chamber (120a);
a second electrolysis step of producing formic acid and an alkali metal or alkaline earth metal electrolyte from the formate produced in the first electrolysis step, wherein the second electrolysis step is carried out in a second diaphragm electrolytic bath (200a) of the apparatus for producing formic acid, wherein the second diaphragm electrolytic bath (200a) includes a second cathode chamber (230a) and a second anode chamber (220a);
wherein a formate solution formed in the first cathode chamber (130a) of the first diaphragm electrolytic bath (100a) is supplied to the second anode chamber (220a) of the second diaphragm electrolytic bath (200a); and
wherein an alkali metal or alkaline earth metal electrolyte solution formed in the second cathode chamber (230a) is supplied in a separate manner to each of the first anode chamber (120a) and the first cathode chamber (130a) of the first diaphragm electrolytic bath (100a) or in a divided manner to both of the first anode chamber (120a) and the first cathode chamber (130a) of the first diaphragm electrolytic bath (100a).

2. The method of claim 1, wherein the first electrolysis step comprises:
generating oxygen (O₂) and a hydrogen ion (H⁺) by subjecting water (H₂O) supplied to the first anode chamber (120a) of the first diaphragm electrolytic bath (100a) to an electrolysis reaction at a first anode (121a);
transferring the hydrogen ion (H⁺) generated at the first anode (121a) to the first cathode chamber (130a) through a first diaphragm (110a);
converting carbon dioxide supplied to the first cathode chamber (130a) into a formic acid ion (HCOO⁻) through an electrolysis reaction at a first cathode (131a); and
forming water (H₂O) by combining a hydroxide ion (OH-) of an alkali metal or alkaline earth metal hydroxide electrolyte supplied to the first cathode chamber (130a) with the hydrogen ion (H⁺) passed through the first diaphragm (110a), and forming the formate by combining an alkali metal or alkaline earth metal cation with the formic acid ion (HCOO-); or wherein the first electrolysis step comprises:
generating oxygen (O₂) and a hydrogen ion (H⁺) by subjecting water (H₂O) supplied to the first anode chamber (120a) of the first diaphragm electrolytic bath (100a) to an electrolysis reaction at the first anode (121a);
forming an acid or water by combining the hydrogen ion (H⁺) generated at the first anode (121a) with an anion of the alkali metal or alkaline earth metal electrolyte supplied to the first anode chamber (120a);
transferring an alkali metal or alkaline earth metal cation remaining after formation of the acid or water to the first cathode chamber (130a) through the first diaphragm (110a);
converting carbon dioxide supplied to the first cathode chamber (130a) into a formic acid ion (HCOO⁻) through an electrolysis reaction at the first cathode (131a); and
forming the formate by combining, in the first cathode chamber (130a), the alkali metal or alkaline earth metal cation transferred through the first diaphragm (110a) with the formic acid ion (HCOO⁻) generated at the first cathode (131a).

3. The method of claim 1, wherein the second electrolysis step comprises:
supplying a formate solution to the second anode chamber (220a) of the second diaphragm electrolytic bath (200a);
generating oxygen (O₂) and a hydrogen ion (H⁺) by subjecting water (H₂O) of the formate solution supplied to the second anode chamber (220a) to an electrolysis reaction at a second anode (221a);
forming formic acid (HCOOH) by reacting the hydrogen ion (H⁺) generated at the second anode (221a) with a formic acid ion (HCOO⁻) of the formate solution;
transferring an alkali metal or alkaline earth metal cation, remaining after formation of formic acid, to the second cathode chamber (230a) through a second diaphragm (210a);
converting water into hydrogen (H₂) and a hydroxide ion (OH-) through an electrolysis reaction at a second cathode (231a) of the second cathode chamber (230a); and
forming the alkali metal or alkaline earth metal electrolyte through reaction of the converted hydroxide ion (OH-) with a solution supplied to the second cathode chamber (230a) and the alkali metal or alkaline earth metal cation transferred through the second diaphragm (210a).

4. The method of claim 1, wherein the method further comprises:
supplying water discharged from the first anode chamber (120a) of the first diaphragm electrolytic bath (100a) to the second cathode chamber (230a) of the second diaphragm electrolytic bath (200a).

## Patentansprüche

1. Verfahren zur Herstellung von Ameisensäure durch eine elektrische Reduktion von Kohlendioxid, wobei das Verfahren umfasst:
einen ersten Elektrolyseschritt zur Herstellung eines Formiats aus zugeführtem Kohlendioxid, wobei der erste Elektrolyseschritt in einem ersten Diaphragma-Elektrolysebad (100a) einer Vorrichtung zur Herstellung von Ameisensäure durchgeführt wird, wobei das erste Diaphragma-Elektrolysebad (100a) eine erste Kathodenkammer (130a) und eine erste Anodenkammer (120a) aufweist;
einen zweiten Elektrolyseschritt zur Herstellung von Ameisensäure und eines Alkalimetall- oder Erdalkalimetallelektrolyten aus dem in dem ersten Elektrolyseschritt hergestellten Formiat, wobei der zweite Elektrolyseschritt in einem zweiten Diaphragma-Elektrolysebad (200a) der Vorrichtung zur Herstellung von Ameisensäure durchgeführt wird, wobei das zweite Diaphragma-Elektrolysebad (200a) eine zweite Kathodenkammer (230a) und eine zweite Anodenkammer (220a) aufweist;
wobei eine Formiatlösung, die in der ersten Kathodenkammer (130a) des ersten Diaphragma-Elektrolysebades (100a) gebildet worden ist, der zweiten Anodenkammer (220a) des zweiten Diaphragma-Elektrolysebades (200a) zugeführt wird; und
wobei eine Alkalimetall- oder Erdalkalimetall-Elektrolytlösung, die in der zweiten Kathodenkammer (230a) gebildet worden ist, auf eine getrennte Weise zu jeder von der ersten Anodenkammer (120a) und von der ersten Kathodenkammer (130a) des ersten Diaphragma-Elektrolysebades (100a) oder auf eine geteilte Weise sowohl der ersten Anodenkammer (120a) als auch der ersten Kathodenkammer (130a) des ersten Diaphragma-Elektrolysebades (100a) zugeführt wird.

2. Verfahren nach Anspruch 1, wobei der erste Elektrolyseschritt umfasst:
ein Erzeugen von Sauerstoff (O₂) und eines Wasserstoffions (H⁺), indem das Wasser (H₂O), das der ersten Anodenkammer (120a) des ersten Diaphragma-Elektrolysebades (100a) zugeführt worden ist, einer Elektrolysereaktion an einer ersten Anode (121a) unterzogen wird;
ein Übertragen des Wasserstoffions (H⁺), das an der ersten Anode (121a) erzeugt worden ist, an die erste Kathodenkammer (130a) durch ein erstes Diaphragma (110a);
ein Umwandeln von Kohlendioxid, das der ersten Kathodenkammer (130a) zugeführt worden ist, in ein Ameisensäureion (HCOO⁻) durch eine Elektrolysereaktion an einer ersten Kathode (131a); und
ein Bilden von Wasser (H₂O) durch ein Kombinieren eines Hydroxidions (OH⁻) eines Alkalimetall- oder Erdalkalimetall-Hydroxid-Elektrolyten, der der ersten Kathodenkammer (130a) zugeführt worden ist, mit dem Wasserstoffion (H⁺), das durch das erste Diaphragma (110a) hindurchgegangen ist, und ein Bilden des Formiats durch ein Kombinieren eines Alkalimetall- oder Erdalkalimetall-Kations mit dem AmeisensäureIon (HCOO-); oder wobei der erste Elektrolyseschritt umfasst:
ein Erzeugen von Sauerstoff (O₂) und eines Wasserstoffions (H+), indem das Wasser (H₂O), das der ersten Anodenkammer (120a) des ersten Diaphragma-Elektrolysebades (100a) zugeführt worden ist, einer Elektrolysereaktion an der ersten Anode (121a) unterzogen wird;
ein Bilden einer Säure oder von Wasser durch ein Kombinieren des Wasserstoffions (H⁺), das an der ersten Anode (121a) erzeugt worden ist, mit einem Anion des Alkali- oder Erdalkalielektrolyten, das der ersten Anodenkammer (120a) zugeführt worden ist;
ein Übertragen eines Alkalimetall- oder Erdalkalimetallkations, das nach der Bildung der Säure oder des Wassers verblieben ist, an die erste Kathodenkammer (130a) durch das erste Diaphragma (110a);
ein Umwandeln des Kohlendioxids, das der ersten Kathodenkammer (130a) zugeführt worden ist, in ein Ameisensäureion (HCOO⁻) durch eine Elektrolysereaktion an der ersten Kathode (131a); und
ein Bilden des Formiats in der ersten Kathodenkammer (130a) durch ein Kombinieren des Alkali- oder Erdalkalimetallkations, das durch das erste Diaphragma (110a) übertragen worden ist, mit dem Ameisensäureion (HCOO-), das an der ersten Kathode (131a) erzeugt worden ist.

3. Verfahren nach Anspruch 1, wobei der zweite Elektrolyseschritt umfasst:
ein Zuführen einer Formiatlösung in die zweite Anodenkammer (220a) des zweiten Diaphragma-Elektrolysebades (200a);
ein Erzeugen von Sauerstoff (O₂) und eines Wasserstoffions (H⁺), indem das Wasser (H₂O) der Formiatlösung, das der zweiten Anodenkammer (220a) zugeführt worden ist, einer Elektrolysereaktion an einer zweiten Anode (221a) unterzogen wird;
ein Bilden von Ameisensäure (HCOOH), indem das Wasserstoffion (H⁺), das an der zweiten Anode (221a) erzeugt worden ist, mit einem Ameisensäureion (HCOO-) der Formiatlösung reagiert wird;
ein Übertragen eines Alkalimetall- oder Erdalkalimetallkations, das nach der Bildung der Ameisensäure verblieben ist, in die zweite Kathodenkammer (230a) durch ein zweites Diaphragma (210a);
ein Umwandeln von Wasser in Wasserstoff (H₂) und ein Hydroxidion (OH⁻) durch eine Elektrolysereaktion an einer zweiten Kathode (231a) der zweiten Kathodenkammer (230a); und
ein Bilden des Alkalimetall- oder Erdalkalimetallelektrolyten durch eine Reaktion des umgewandelten Hydroxidions (OH⁻) mit einer Lösung, die der zweiten Kathodenkammer (230a) zugeführt worden ist, und dem Alkalimetall- oder Erdalkalimetallkation, das durch das zweite Diaphragma (210a) übertragen worden ist.

4. Verfahren nach Anspruch 1, wobei das Verfahren ferner umfasst:
ein Zuführen von Wasser, das aus der ersten Anodenkammer (120a) des ersten Diaphragma-Elektrolysebads (100a) abgelassen worden ist, in die zweite Kathodenkammer (230a) des zweiten Diaphragma-Elektrolysebads (200a).

## Revendications

1. Procédé de production d'acide formique par le biais d'une réduction électrique de dioxyde de carbone, comprenant :
une première étape d'électrolyse consistant à produire un formiate à partir de dioxyde de carbone qui est fourni, dans lequel la première étape d'électrolyse est réalisée dans un premier bain électrolytique à membrane (100a) d'un appareil pour produire de l'acide formique, dans lequel le premier bain électrolytique à membrane (100a) comporte une première chambre de cathode (130a) et une première chambre d'anode (120a) ;
une seconde étape d'électrolyse consistant à produire de l'acide formique et un électrolyte de métal alcalin ou de métal alcalino-terreux à partir du formiate produit dans la première étape d'électrolyse, dans lequel la seconde étape d'électrolyse est réalisée dans un second bain électrolytique à membrane (200a) de l'appareil pour produire de l'acide formique, dans lequel le second bain électrolytique à membrane (200a) comporte une seconde chambre de cathode (230a) et une seconde chambre d'anode (220a) ;
dans lequel une solution de formiate formée dans la première chambre de cathode (130a) du premier bain électrolytique à membrane (100a) est fournie à la seconde chambre d'anode (220a) du second bain électrolytique à membrane (200a) ; et
dans lequel une solution d'électrolyte de métal alcalin ou de métal alcalino-terreux formée dans la seconde chambre de cathode (230a) est fournie d'une manière séparée à chacune de la première chambre d'anode (120a) et de la première chambre de cathode (130a) du premier bain électrolytique à membrane (100a) ou d'une manière divisée à la fois à la première chambre d'anode (120a) et à la première chambre de cathode (130a) du premier bain électrolytique à membrane (100a).

2. Procédé selon la revendication 1, dans lequel la première étape d'électrolyse comprend :
la génération d'oxygène (O₂) et d'un ion hydrogène (H⁺) par soumission de l'eau (H₂O) fournie à la première chambre d'anode (120a) du premier bain électrolytique à membrane (100a) à une réaction d'électrolyse au niveau d'une première anode (121a) ;
le transfert de l'ion hydrogène (H⁺) généré au niveau de la première anode (121a) vers la première chambre de cathode (130a) à travers une première membrane (110a) ;
la conversion de dioxyde de carbone fourni à la première chambre de cathode (130a) en un ion acide formique (HCOO⁻) par le biais d'une réaction d'électrolyse au niveau d'une première cathode (131a) ; et
la formation d'eau (H₂O) par combinaison d'un ion hydroxyde (OH⁻) d'un électrolyte d'hydroxyde de métal alcalin ou de métal alcalino-terreux fourni à la première chambre de cathode (130a) avec l'ion hydrogène (H⁺) passé à travers la première membrane (110a), et la formation du formiate par combinaison d'un cation de métal alcalin ou de métal alcalino-terreux avec l'ion acide formique (HCOO⁻) ; ou dans lequel la première étape d'électrolyse comprend :
la génération d'oxygène (O₂) et d'un ion hydrogène (H⁺) par soumission de l'eau (H₂O) fournie à la première chambre d'anode (120a) du premier bain électrolytique à membrane (100a) à une réaction d'électrolyse au niveau de la première anode (121a) ;
la formation d'un acide ou d'eau par combinaison de l'ion hydrogène (H⁺) généré au niveau de la première anode (121a) avec un anion de l'électrolyte de métal alcalin ou de métal alcalino-terreux fourni à la première chambre d'anode (120a) ;
le transfert d'un cation de métal alcalin ou de métal alcalino-terreux restant après formation de l'acide ou d'eau vers la première chambre de cathode (130a) à travers la première membrane (110a) ;
la conversion de dioxyde de carbone fourni à la première chambre de cathode (130a) en un ion acide formique (HCOO⁻) par le biais d'une réaction d'électrolyse au niveau de la première cathode (131a) ; et
la formation du formiate par combinaison, dans la première chambre de cathode (130a), du cation de métal alcalin ou de métal alcalino-terreux transféré à travers la première membrane (110a) avec l'ion acide formique (HCOO⁻) généré au niveau de la première cathode (131a).

3. Procédé selon la revendication 1, dans lequel la seconde étape d'électrolyse comprend :
la fourniture d'une solution de formiate vers la seconde chambre d'anode (220a) du second bain électrolytique à membrane (200a) ;
la génération d'oxygène (O₂) et d'un ion hydrogène (H⁺) par soumission de l'eau (H₂O) de la solution de formiate fournie à la seconde chambre d'anode (220a) à une réaction d'électrolyse au niveau d'une seconde anode (221a) ;
la formation d'acide formique (HCOOH) par réaction de l'ion hydrogène (H⁺) généré au niveau de la seconde anode (221a) avec un ion acide formique (HCOO⁻) de la solution de formiate ;
le transfert d'un cation de métal alcalin ou de métal alcalino-terreux, restant après formation d'acide formique, vers la seconde chambre de cathode (230a) à travers une seconde membrane (210a) ;
la conversion de l'eau en hydrogène (H₂) et en un ion hydroxyde (OH⁻) par le biais d'une réaction d'électrolyse au niveau d'une seconde cathode (231a) de la seconde chambre de cathode (230a) ; et
la formation de l'électrolyte de métal alcalin ou de métal alcalino-terreux par réaction de l'ion hydroxyde (OH⁻) converti avec une solution fournie à la seconde chambre de cathode (230a) et du cation de métal alcalin ou de métal alcalino-terreux transféré à travers la seconde membrane (210a).

4. Procédé selon la revendication 1, dans lequel le procédé comprend en outre :
la fourniture de l'eau évacuée à partir de la première chambre d'anode (120a) du premier bain électrolytique à membrane (100a) vers la seconde chambre de cathode (230a) du second bain électrolytique à membrane (200a).
